# FASCICULE DE BREVET EUROPEEN

(11) **EP 3 304 043 B1**
(45) Date de publication et mention de la délivrance du brevet: **09.03.2022**
(21) Numéro de dépôt: 15730211.8
(22) Date de dépôt: 02.06.2015
(51) Int. Cl.: G01N 21/17, G01N 21/49, G02F 1/11, A61B 5/00

(54) **PROCEDES ET SYSTEMES D'IMAGERIE ACOUSTO-OPTIQUE**
VERFAHREN UND SYSTEME ZUM AKUSTO-OPTISCHEN ABBILDEN
METHODS AND SYSTEMS FOR ACOUSTO-OPTICAL IMAGING

(43) Date de publication de la demande: 11.04.2018
(73) Titulaire: Centre National de la Recherche Scientifique (CNRS), 75016 Paris (FR); INSERM - Institut National de la Santé et de la Recherche Médicale, 75013 Paris (FR)
(72) Inventeur: RAMAZ, François, 77120 Coulommiers (FR); TANTER, Mickaël, 92220 Bagneux (FR); LAUDEREAU, Jean-Baptiste, 75020 Paris (FR); GENNISSON, Jean-Luc, 95000 Cergy (FR)
(74) Mandataire: Osha Liang
(86) Numéro de dépôt international: PCT/FR2015/051448
(87) Numéro de publication internationale: WO 2016/193554

(56) Documents cités:
- FR-A1- 2 617 602
- US-A- 6 041 248
- US-A1- 2008 296 514
- WANG L V ET AL: "FREQUENCY-SWEPT ULTRASOUND-MODULATED OPTICAL TOMOGRAPHY OF SCATTERING MEDIA", OPTICS LETTERS, OPTICAL SOCIETY OF AMERICA, US, vol. 23, no. 12, 15 juin 1998 (1998-06-15) , pages 975-977, XP000766613, ISSN: 0146-9592
- KILICASLAN I ET AL: "Ultrasonic enhanced Brillouin light scattering in water", OPTICS COMMUNICATIONS, NORTH-HOLLAND PUBLISHING CO. AMSTERDAM, NL, vol. 265, no. 2, 15 septembre 2006 (2006-09-15), pages 441-445, XP028081221, ISSN: 0030-4018, DOI: 10.1016/J.OPTCOM.2006.03.061 [extrait le 2006-09-15]
- KEMPE M ET AL: "ACOUSTO-OPTIC TOMOGRAPHY WITH MULTIPLY SCATTERED LIGHT", JOURNAL OF THE OPTICAL SOCIETY OF AMERICA A, OPTICAL SOCIETY OF AMERICA, US, vol. 14, no. 5, 1 mai 1997 (1997-05-01), pages 1151-1158, XP000997070, ISSN: 1084-7529

## Description

La présente invention est relative aux procédés et aux systèmes d'imagerie acousto-optique.

Plus particulièrement, l'invention se rapporte notamment à un procédé d'imagerie acousto-optique pour imager une zone d'observation d'un milieu.

Le document brevet US 2008/296514 A1 décrit des procédés et dispositifs de mesures acousto-optiques dans lesquels des rayonnements électromagnétiques sont marqués (« taggés ») par des ondes acoustiques se propageant dans un milieu.

Un tel procédé vise à obtenir, de manière non-invasive, une information sur les propriétés optiques d'une zone d'observation située en profondeur dans un milieu, par exemple des tissus biologiques. Les propriétés optiques peuvent être par exemple une couleur, une absorption, ou encore une structure des tissus biologiques de la zone d'observation. La zone d'observation est par exemple située à quelques millimètres ou centimètres en profondeur dans un objet, par exemple à l'intérieur d'un corps, d'un organe ou d'un objet.

On connait de tels procédés, dans lesquels on génère, dans la zone d'observation du milieu, une onde acoustique ultrasonore focalisée sur une tache focale dans la zone d'observation et on émet simultanément une onde lumineuse dans cette même zone. On obtient alors une information en détectant un signal lié au couplage entre l'onde lumineuse et la vibration acoustique dans le milieu. En effet, lorsqu'une onde ultrasonore, de fréquence acoustique fa traverse un milieu diffusant (par exemple un tissu biologique ou autre), elle provoque un déplacement périodique des diffuseurs et une modulation périodique de l'indice de réfraction du milieu. Si une onde lumineuse incidente, notamment une onde laser, de fréquence incidente fi est diffusée par le milieu, le mouvement des diffuseurs et la modulation de l'indice de réfraction du milieu génèrent une onde lumineuse marquée comprenant d'une part une composante porteuse à la fréquence incidente fi , et d'autre part une composante acousto-optique diffusée sur l'une ou l'autre des bandes latérales acoustiques, de fréquences **fao = fa ±** n ∗ **fi.**

De tels procédés sont notamment décrits dans « Ultrasound-mediated optical tomography: a review of current methods » de Daniel S. Elson, Rui Li, Christopher Dunsby, Robert Eckersley et Meng-Xing Tang, publié dans Interface Focus (2011) vol. 1, pages 632-648.

Dans ces procédés connus, on obtient alors l'information sur le milieu en déterminant le poids de la composante acousto-optique par rapport à la composante porteuse. Par ailleurs, l'onde lumineuse marquée provient de la tache focale de l'onde ultrasonore, il est donc possible d'obtenir une information sur l'intensité lumineuse locale dans le milieu diffusant. Dans ces procédés, on forme alors, ligne par ligne, une image de l'intensité lumineuse à l'intérieur de la zone d'observation en balayant la zone d'observation avec une succession d'ondes ultrasonores focalisées.

Ces procédés connus présentent notamment l'inconvénient de nécessiter l'émission d'un grand nombre d'onde ultrasonores pour obtenir une image de la zone d'observation et donc d'être lents. Par ailleurs, le rapport signal sur bruit est faible et impose de réaliser un grand nombre d'acquisitions au même point focal pour les moyenner et obtenir un signal exploitable.

Pour pallier ces inconvénients, l'invention a pour premier objet un procédé d'imagerie acousto-optique pour imager une zone d'observation d'un milieu selon la revendication 1.

Dans des modes de réalisation préférés de l'invention, on peut éventuellement avoir recours en outre à l'une et/ou à l'autre des dispositions suivantes :
- l'onde acoustique non-focalisée est une onde acoustique plane ;
- l'onde acoustique non-focalisée est une onde acoustique divergente ;
- les directions de propagation de la pluralité d'ondes acoustiques non-focalisées couvrent un secteur angulaire d'angle supérieur à 10 degrés ;
- les directions de propagation de la pluralité d'ondes acoustiques non-focalisées sont séparées par un pas angulaire supérieur à 0.1 degré, de préférence supérieur à 0.5 degré ;
- les directions de propagation de la pluralité d'ondes acoustiques non-focalisées sont séparées par un pas angulaire inférieur à 45 degrés, de préférence inférieur à 20 degrés ; inférieur à 45 degrés, de préférence inférieur à 20 degrés ;
- chaque signal de mesure associée à une onde acoustique non-focalisée comporte une série temporelle de valeurs d'intensité lumineuse contenant de l'information relative à la composante acousto-optique de l'onde lumineuse marquée décalée en fréquence par l'onde acoustique non-focalisée ;
- chaque signal de mesure associée à une onde acoustique non-focalisée est échantillonné à une fréquence supérieure à 2 mégahertz, de préférence supérieur à dix mégahertz ;
- l'étape de traitement comprend la mise en œuvre d'une transformation de Radon inverse ;
- l'étape de traitement comprend la mise en œuvre d'un algorithme de formation de voies ;
- l'étape de traitement comprend la mise en œuvre d'un algorithme de rétroprojection ou de rétroprojection filtrée ;
- une opération de mesure est réitérée *n* fois pour acquérir *n* signaux de mesure associés à une direction de propagation d'une onde acoustique non-focalisée, et lesdits *n* signaux de mesure sont moyennés ensembles pour déterminer un signal de mesure associé à ladite onde acoustique non-focalisée utilisé pour l'étape de traitement ;
- l'opération de mesure est réitérée un nombre *n* de fois supérieur ou égal à 1.

L'invention a également pour objet un système d'imagerie acousto-optique d'une zone d'observation d'un milieu selon la revendication 15.

D'autres caractéristiques et avantages de l'invention apparaîtront au cours de la description suivante de plusieurs de ses formes de réalisation, données à titre d'exemples non limitatifs, en regard des dessins joints.

Sur les dessins :
- la figure 1 est une représentation schématique d'un système selon un mode de réalisation de l'invention,
- la figure 2 est un organigramme schématique d'un procédé selon un mode de réalisation de l'invention,
- les figures 3A et 3B illustrent des détails d'un mode de réalisation des opérations d'une étape de traitement du procédé de la figure 2,
- la figure 4 illustre des valeurs représentatives d'intensité lumineuse obtenues lors de la mise en œuvre d'un mode de réalisation de l'invention et lors de la mise en œuvre d'un procédé de l'art antérieur de l'invention.

Sur les différentes figures, les mêmes références désignent des éléments identiques ou similaires.

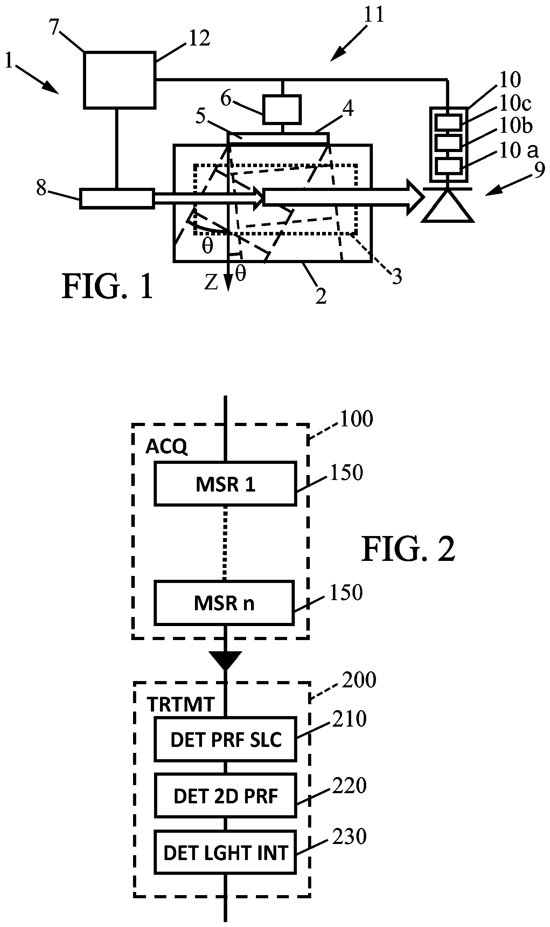

La figure 1 représente schématiquement un système d'imagerie acousto-optique 1 selon un mode de réalisation de l'invention.

On dispose ainsi d'un milieu 2, par exemple un objet ou un tissu biologique, à imager et comportant donc une zone d'observation 3. La zone d'observation 3 peut être en surface du milieu 2 mais peut également être localisée en profondeur dans le milieu 2, par exemple à quelques centimètres de profondeur. Le milieu 2 est un milieu diffusant. Par « milieu diffusant », on entend notamment qu'au-delà d'une épaisseur caractéristique 1* (libre parcours moyen de transport), qui est par exemple de l'ordre du millimètre dans les milieux biologiques, l'information que contient une onde lumineuse traversant le milieu est totalement brouillée et impossible à interpréter sans traitement. Ceci rend donc impossible une imagerie optique classique en profondeur. Ce phénomène est également appelé diffusion multiple de la lumière.

Un réseau de transducteurs acoustiques 4 est en contact acoustique avec le milieu 2, soit directement en contact, soit par exemple couplé acoustiquement au milieu 2 par l'intermédiaire d'un élément de couplage comme une cuve ou un coussin rempli d'eau.

Le réseau de transducteurs acoustiques 4 est par exemple un réseau linéaire comportant par exemple quelques dizaines de transducteurs 5 (par exemple de 100 à 300). Les transducteurs 5 sont par exemple juxtaposés selon un axe X. Dans des variantes de réalisation, les transducteurs 5 pourront également être disposés en suivant une courbe, ou encore arrangés pour former une matrice bidimensionnelle.

Le réseau de transducteurs acoustiques 4 est commandé par des moyens de commande qui comportent par exemple une baie électronique 6 et un micro-ordinateur 7 contrôlant la baie électronique 6.

Le réseau de transducteurs acoustiques 4 est ainsi apte à générer, dans la zone d'observation 3, une onde acoustique non-focalisée se propageant selon une direction de propagation prédéfinie. La direction de propagation peut être contrôlée de sorte à générer dans la zone d'observation 3 des ondes acoustiques non-focalisées se propageant selon des directions de propagation variées.

A titre non limitatif, le réseau de transducteurs acoustiques 4 est par exemple apte à générer dans la zone d'observation 3 une onde ultrasonore ayant une fréquence centrale de l'ordre de quelques mégahertz, par exemple 8 MHz. Le réseau de transducteurs acoustiques 4 est par exemple apte à générer dans la zone d'observation 3 une pluralité d'ondes ultrasonores ayant des directions de propagation choisies dans un secteur angulaire d'angle supérieur à 30 degrés, par exemple de 40 degrés.

Dans un mode de réalisation de l'invention, les ondes acoustiques non-focalisées sont des ondes acoustiques planes. Dans un autre mode de réalisation, les ondes acoustiques non-focalisées sont des ondes acoustiques divergentes.

Le système 1 comporte également un dispositif d'émission lumineuse 8. Le dispositif d'émission lumineuse 8 est apte à émettre dans la zone d'observation 3 au moins une onde lumineuse incidente. En particulier, le dispositif d'émission lumineuse 8 est apte à l'émettre ladite onde lumineuse de manière simultanée avec l'émission d'une onde ultrasonore par le réseau de transducteurs acoustiques 4. Le dispositif d'émission lumineuse 8 est par exemple un laser ou de manière générale un dispositif d'émission permettant de contrôler le spectre de l'onde lumineuse incidente émise.

Par « onde lumineuse », on entend de manière large un rayonnement électromagnétique apte à se propager dans le milieu 2. On peut en particulier entendre un rayonnement électromagnétique appartenant au spectre infrarouge, visible ou ultraviolet.

Dans un exemple fourni à titre purement indicatif et non limitatif, le dispositif d'émission lumineuse 8 est un laser à semi-conducteur monofréquence amplifié de puissance 2 Watts et longueur d'onde 780 nanomètres (ce qui correspond donc à une fréquence incidente fi). La polarisation de l'onde lumineuse incidente peut également être contrôlée. Dans certains modes de réalisation, l'onde lumineuse peut être modulée temporellement et spatialement ou filtrée avant de pénétrer dans le milieu 2.

Le système 1 comporte encore un détecteur 9 apte à acquérir des signaux de mesure représentatifs des ondes lumineuses marquées. Le détecteur 9 est ainsi un photodétecteur sensible à une ou plusieurs longueurs d'onde électromagnétique correspondant(es) à des longueurs d'onde de l'onde lumineuse marquée. Ainsi par exemple, le détecteur 9 est sensible à une composante acousto-optique générée par une interaction entre une onde lumineuse incidente et une onde acoustique non-focalisée se propageant dans la zone d'observation. Le détecteur 9 peut également être sensible à une composante porteuse, c'est-à-dire une composante de l'onde lumineuse marquée à la fréquence incidente fi.

Le détecteur 9 est par exemple une photodiode.

Le système 1 peut comporter des éléments de pré traitement ou de post traitement du signal 10, éventuellement intégrés dans le détecteur 9. Les éléments de post traitement du signal 10 peuvent par exemple comprendre un filtre passe-haut 10a, un amplificateur large bande 10b (par exemple Thorlabs, DHPVA) et un convertisseur analogique numérique 10c.

Ainsi en particulier, le signal de mesure peut être échantillonné par le convertisseur analogique numérique 10c à une fréquence supérieure à quelques mégahertz, de préférence supérieur à dix mégahertz, par exemple une fréquence d'échantillonnage de 40 MHz.

De cette manière, chaque signal de mesure peut notamment comporter une série temporelle de valeurs d'intensité lumineuse d'une composante acousto-optique d'une onde lumineuse marquée décalée en fréquence par une onde acoustique non-focalisée.

Le réseau de transducteurs 4, le dispositif d'émission lumineuse 8 et le détecteur 9 peuvent ainsi former un dispositif d'acquisition 11 d'un système 1 selon l'invention. Un tel dispositif d'acquisition 11 est notamment apte à acquérir une pluralité de signaux de mesure associée à une pluralité d'ondes acoustiques non-focalisées comme cela va être détaillé plus avant ci-après.

Enfin, le système 1 selon l'invention comporte également un dispositif de traitement 12, apte à déterminer au moins une valeur représentative d'une intensité lumineuse dans la zone d'observation 3 à partir de la pluralité de signaux de mesure acquise par le dispositif d'acquisition 11.

Un procédé d'imagerie acousto-optique d'une zone d'observation d'un milieu est notamment illustré plus en détail sur la figure 2 et peut par exemple être mis en œuvre, au moyen du système 1, de la manière suivante.

Au cours d'une étape d'acquisition 100, on acquiert une pluralité de signaux de mesure S associée à une pluralité d'ondes acoustiques non-focalisées se propageant selon des ections de propagation non-colinéaires.

Une telle étape d'acquisition 100 peut comporter au moins une opération de mesure 150, permettant d'acquérir un signal de mesure S associé à une onde acoustique non-focalisée, et en particulier à une direction de propagation θ d'une onde acoustique non-focalisée.

Selon l'invention, étape d'acquisition 100 comporte une pluralité d'opérations de mesure 150 telle que, au cours de chaque opération de mesure de la pluralité d'opérations de mesure on acquiert une pluralité de signaux de mesure (S) associée à ladite pluralité d'ondes acoustiques non-focalisées.

Ainsi au cours de chaque opération de mesure 150 :
- on génère, dans la zone d'observation 3, une onde acoustique non-focalisée se propageant selon une direction de propagation θ au moyen du réseau de transducteurs 4,
- on émet, dans la zone d'observation 3, une onde lumineuse incidente pour générer une onde lumineuse marquée comportant au moins une composante acousto-optique décalée en fréquence par l'onde acoustique non-focalisée au moyen du dispositif d'émission lumineuse 8, et
- on acquière un signal de mesure S représentatif de l'onde lumineuse marquée au moyen du détecteur 9.

En particulier, la pluralité d'opérations de mesure 150 de l'étape d'acquisition 100 est telle que les ondes acoustiques non-focalisées de la pluralité d'ondes acoustiques non-focalisées se propagent selon des directions de propagation non-colinéaires.

Plus précisément, les directions de propagation θ de la pluralité d'ondes acoustiques non-focalisées peuvent couvrir un secteur angulaire d'angle supérieur à 30 degrés. Le secteur angulaire peut être d'angle supérieur à 60 degrés ou 90 degrés, voire proche de 180 degrés.

Dans un mode de réalisation, les directions de propagation θ de la pluralité d'ondes acoustiques non-focalisées sont séparées, les unes des autres, par un pas angulaire supérieur à 0.25 degrés, de préférence supérieur à 0.5 degrés.

Par ailleurs, les directions de propagation θ de la pluralité d'ondes acoustiques non-focalisées peuvent être séparées par un pas angulaire inférieur à 45 degrés, de préférence inférieur à 20 degrés.

Ainsi à titre d'exemple non limitatif, l'étape d'acquisition 100 peut comporter l'émission d'ondes planes selon quarante-et-une directions de propagation θ différentes, lesdites directions de propagation ayant des angles de propagation compris entre -20° et 20° par rapport à une direction Z normale au réseau de transducteurs 4, par pas de 1°. Les ondes planes peuvent par exemple être émises par un système « Aixplorer » ^{®} de la société Supersonic Imagine.

Dans un mode de réalisation de l'invention, une opération de mesure 150 selon une direction de propagation peut être réitérée n fois pour acquérir n signaux de mesure S associés à une direction de propagation θ d'une onde acoustique non-focalisée. L'opération de mesure 150 peut par exemple être réitérée un nombre n de fois supérieur à dix, par exemple une centaine de fois ou un millier de fois.

Une telle réitération de l'opération de mesure 150 permet moyenner ensembles lesdits n signaux de mesure de sorte à déterminer un signal de mesure S à utiliser pour l'étape ultérieure de traitement. Un tel signal de mesure présente l'intérêt d'avoir un rapport signal sur bruit plus élevé.

Le procédé comporte par la suite une étape de traitement 200 au cours de laquelle on détermine au moins une valeur représentative d'une intensité lumineuse dans la zone d'observation à partir de la pluralité de signaux de mesure.

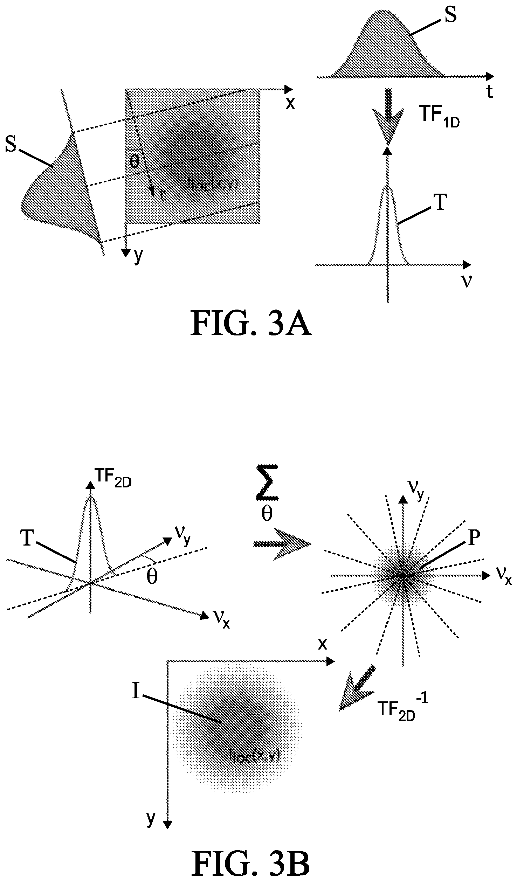

A titre non limitatif, Cette étape de traitement 200 comprend avantageusement la mise en œuvre d'une transformation de Radon comme illustré sur les figures 3A et 3B. L'étape de traitement peut également comporter une double transformée de Fourier (temporelle, puis spatiale).

Selon l'invention, l'étape de traitement 200 comprend les opérations suivantes :
- déterminer (210) une pluralité de tranches de profil associée à la pluralité de signaux de mesure (figure 3A),
- déterminer (220) un profil bidimensionnel à partir de la pluralité de tranches de profil (figure 3B), et
- déterminer (230) au moins une valeur représentative d'une intensité lumineuse dans la zone d'observation à partir du profil bidimensionnel (également figure 3B).

Plus précisément, on commence par déterminer, pour chaque signal de mesure S obtenu au cours de l'étape de traitement, une tranche de profil T.

Pour cela, on met en œuvre une transformation de Fourier unidimensionnelle du signal de mesure S qui fournit la tranche de profil T associée comme illustré sur la figure 3A. On comprend donc bien que la qualité de l'échantillonnage du signal de mesure S est importante pour garantir une conservation des informations lors de la transformation de Fourier.

Puis, à partir de la pluralité de tranches de profil T associées à la pluralité de signaux de mesure S, on détermine un profil bidimensionnel P. Comme cela est illustré sur la figure 3B, le profil bidimensionnel P est déterminé par recalage dans un espace de Fourier de la pluralité de tranches de profil. Chaque tranche de profil T est ainsi recalée dans l'espace de Fourier en fonction de la direction de propagation θ de l'onde acoustique non-focalisée qui était associée au signal de mesure S associé à la tranche de profil T.

Ainsi, il est par exemple possible de recaler les tranches de profil T pour emplir le secteur angulaire constitué par les directions de propagation des ondes non-focalisées.

Une fois le profil bidimensionnel P obtenu, il est alors possible de déterminer une ou plusieurs valeur(s) représentative(s) d'une intensité lumineuse I dans la zone d'observation 3 par une transformation de Fourier bidimensionnelle inverse du profil bidimensionnel P, comme illustré également sur la figure 3B.

Dans des modes de réalisation de l'invention, les tranches de profil T peuvent être complétées pour déterminer le profil bidimensionnel P.

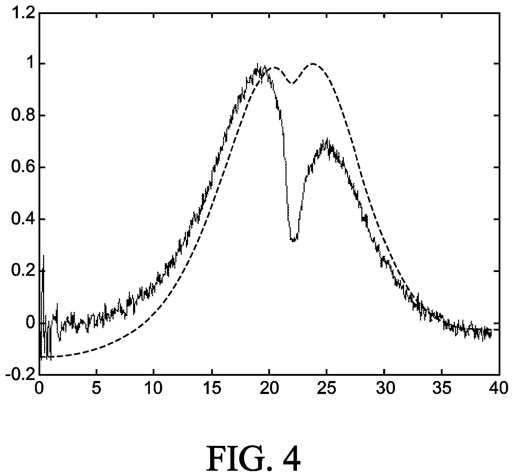

La figure 4 illustre un résultat obtenu lors de la mise en œuvre d'un exemple de réalisation de l'invention. Le graphique de cette figure illustre l'intensité lumineuse en fonction de la position selon un axe traversant la zone d'observation. Dans cette mise en œuvre de l'invention, le milieu imagé est un gel d'agar mélangé à de l'intralipid 10%. Un tel gel est très diffusant de telle sorte qu'au-delà d'un millimètre, il est impossible d'observer ce qui se trouve à l'intérieur. Un objet absorbant, ou localement plus ou moins diffusant que le gel, est placé dans le gel diffusant. A titre d'exemple purement indicatif, une gaine de fil électrique noire d'environ 1 mm de diamètre et 5 millimètre de long peut être placée sensiblement au milieu du gel pour obtenir l'exemple de la figure 4.

Le graphique de la figure 4 permet de comparer l'intensité lumineuse en fonction de la position obtenue par une mise en œuvre de l'invention (en traits pointillés) avec l'intensité lumineuse en fonction de la position obtenue par une mise en œuvre d'un procédé connu tel que décrit dans l'introduction de la présente description, dans lequel on balaye la zone d'observation à l'aide d'ondes ultrasonores focalisées (en traits pleins).

Dans les deux mises en œuvre, chaque opération de mesures est répétée 1000 fois pour obtenir un signal de mesure moyenné.

Dans la mise en œuvre de l'invention de la figure 4 (traits pointillés), des ondes planes comprises entre -20° et 20° par pas de 1° ont été émises par le réseau de transducteur 4.

Dans la mise en œuvre du procédé connu de la figure 4, les valeurs d'intensité lumineuse sont obtenues en réalisant une série d'émission d'ondes ultrasonores focalisées dont les taches focales sont décalées les unes des autres de 0.2 mm de sorte à obtenir une image de l'intensité lumineuse dans la zone d'observation 3 avec 100 colonnes.

Comme cela est visible sur la figure 4, le rapport signal-à-bruit (RSB) de l'intensité lumineuse en fonction de la position obtenue par la mise en œuvre de l'invention est bien meilleur que le rapport signal-à-bruit de l'intensité lumineuse en fonction de la position obtenue par la mise en œuvre dudit procédé connu.

Ce résultat surprenant s'explique notamment par le fait que les ondes ultrasonores non-focalisées permettent de coder, dans chaque onde lumineuse marquée, de l'information provenant de tous les points de la zone d'observation. Au contraire, dans le procédé connus, l'utilisation d'ondes ultrasonores focalisées implique que chaque onde lumineuse marquée contient de l'information provenant uniquement de la proximité de la tache focale.

A rapport-signal-à-bruit équivalent, il faut donc plusieurs dizaines de fois moins de d'opérations de mesure avec les ondes acoustiques non-focalisées qu'avec les ondes acoustiques focalisées.

Par rapport à l'exemple présenté, on pourra, lors de variantes de réalisation, mettre en œuvre l'une et/ou l'autre des dispositions suivantes :
- utiliser un laser plus puissant,
- utiliser une photodiode de bruit inférieur, telle qu'une photo diode refroidie par Peltier,
- utiliser des ondes divergentes,
- balayer un secteur angulaire d'angle plus important (tomographie 360 degrés),
- utiliser des ondes acoustiques non-focalisées de plus forte puissance, par exemple d'énergie proche de l'échographie standard.

De cette manière, à rapport-signal-à-bruit équivalent, il est donc envisageable de gagner d'un facteur dix à un facteur cent, voire plus, sur le temps d'imagerie par rapport aux procédés connus décrits dans l'introduction de la présente description.

## Revendications

1. Procédé d'imagerie acousto-optique pour imager une zone d'observation (3) d'un milieu (2), le procédé comprenant
- une étape d'acquisition (100) au cours de laquelle on acquière une pluralité de signaux de mesure (S) associée à une pluralité d'ondes acoustiques non-focalisées se propageant selon des directions de propagation (θ) non-colinéaires, l'étape d'acquisition comportant une pluralité d'opérations de mesure (150) telle que, au cours de chaque opération de mesure (150) de la pluralité d'opérations de mesure (150) :
- on génère, dans la zone d'observation (3), une onde acoustique non-focalisée se propageant selon une direction de propagation (θ) ;
- on émet dans la zone d'observation (3) une onde lumineuse incidente pour générer une onde lumineuse marquée comportant au moins une composante acousto-optique décalée en fréquence par l'onde acoustique non-focalisée ;
- on acquiert un signal de mesure (S), associé à ladite onde acoustique non-focalisée, contenant de l'information à propos de l'onde lumineuse marquée ; et
- une étape de traitement (200) au cours de laquelle on détermine au moins une valeur représentative d'une intensité lumineuse (I) dans la zone d'observation (3) à partir de ladite pluralité de signaux de mesure (S), l'étape de traitement comprenant :
- une détermination (210) d'une pluralité de tranches de profil (T), chaque tranche de profil étant fonction d'une transformation de Fourier unidimensionnelle d'un signal de mesure associé acquis pour une onde acoustique non-focalisée se propageant selon une direction de propagation associée ;
- une détermination d'un spectre bidimensionnel (P) à partir de la pluralité de tranches de profil (T), et
- une détermination de ladite au moins une valeur représentative de l'intensité lumineuse (I) dans la zone d'observation (3), ladite valeur représentative étant fonction d'une transformation de Fourier bidimensionnelle inverse du spectre bidimensionnel (P).

2. Procédé selon la revendication 1, dans lequel l'onde acoustique non-focalisée est une onde acoustique plane.

3. Procédé selon la revendication 1, dans lequel l'onde acoustique non-focalisée est une onde acoustique divergente.

4. Procédé selon l'une quelconque des revendications 1 à 3, dans lequel les directions de propagation (θ) de la pluralité d'ondes acoustiques non-focalisées couvrent un secteur angulaire d'angle supérieur à 10 degrés.

5. Procédé selon l'une quelconque des revendications 1 à 4, dans lequel les directions de propagation (θ) de la pluralité d'ondes acoustiques non-focalisées sont séparées par un pas angulaire supérieur à 0.1 degré, de préférence supérieur à 0.5 degré.

6. Procédé selon l'une quelconque des revendications 1 à 5, dans lequel les directions de propagation (θ) de la pluralité d'ondes acoustiques non-focalisées sont séparées par un pas angulaire inférieur à 45 degrés, de préférence inférieur à 20 degrés.

7. Procédé selon l'une quelconque des revendications 1 à 6, dans lequel chaque signal de mesure (S) associée à une onde acoustique non-focalisée comporte une série temporelle de valeurs d'intensité lumineuse contenant de l'information relative à la composante acousto-optique de l'onde lumineuse marquée décalée en fréquence par l'onde acoustique non-focalisée.

8. Procédé selon l'une quelconque des revendications 1 à 7, dans lequel chaque signal de mesure (S) associée à une onde acoustique non-focalisée est échantillonné à une fréquence supérieure à 2 mégahertz, de préférence supérieur à dix mégahertz.

9. Procédé selon l'une quelconque des revendications 1 à 8, dans lequel l'étape de traitement (200) comprend la mise en œuvre d'une transformation de Radon inverse.

10. Procédé selon l'une quelconque des revendications 1 à 9, dans lequel l'étape de traitement (200) comprend la mise en œuvre d'un algorithme de formation de voies.

11. Procédé selon l'une quelconque des revendications 1 à 10, dans lequel l'étape de traitement (200) comprend la mise en œuvre d'un algorithme de rétroprojection ou de rétroprojection filtrée.

12. Procédé selon l'une quelconque des revendications 1 à 11, dans lequel on détermine le spectre bidimensionnel (P) par recalage dans un espace de Fourier de la pluralité de tranches de profil (T), chaque tranche de profil étant recalée en fonction de la direction de propagation (θ) d'une onde acoustique non-focalisée associée au signal de mesure (S) associé à la tranche de profil (T).

13. Procédé selon l'une quelconque des revendications 1 à 12, dans lequel une opération de mesure (150) est réitérée *n* fois pour acquérir *n* signaux de mesure (S) associés à une direction de propagation (θ) d'une onde acoustique non-focalisée, et dans lequel lesdits n signaux de mesure sont moyennés ensembles pour déterminer un signal de mesure associé à ladite onde acoustique non-focalisée utilisé pour l'étape de traitement (200).

14. Procédé selon l'une quelconque des revendications 1 à 13, dans lequel l'opération de mesure (150) est réitérée un nombre *n* de fois supérieur ou égal à 1.

15. Système (1) d'imagerie acousto-optique d'une zone d'observation (3) d'un milieu (2), le système comprenant
- un dispositif d'acquisition (11) d'une pluralité de signaux de mesure (S) associée à une pluralité d'ondes acoustiques non-focalisées se propageant selon des directions de propagation (θ) non-colinéaires, le dispositif d'acquisition comportant :
- un réseau de transducteurs (4) pour générer, dans la zone d'observation (3), une pluralité d'ondes acoustiques non-focalisées se propageant selon des directions de propagation (θ) non-colinéaires,
- un dispositif d'émission lumineuse (8) pour émettre dans la zone d'observation (3) au moins une onde lumineuse incidente pour générer une pluralité d'ondes lumineuses marquées comportant chacune au moins une composante acousto-optique décalée en fréquence respectivement par une onde acoustique non-focalisée de ladite pluralité d'ondes acoustiques non-focalisées,
- un détecteur (9) pour acquérir une pluralité de signaux de mesure (S) associés à ladite pluralité d'ondes acoustiques non-focalisées et respectivement représentatifs de chaque onde lumineuse marquée de ladite pluralité d'ondes lumineuses marquées; et
- un dispositif de traitement (12) configuré pour déterminer au moins une valeur représentative d'une intensité lumineuse (I) dans la zone d'observation (3) à partir de ladite pluralité de signaux de mesure (S) acquis par le dispositif d'acquisition (11), le dispositif de traitement comprenant des moyens configurés pour :
- déterminer (210) une pluralité de tranches de profil (T), chaque tranche de profil étant fonction d'une transformation de Fourier unidimensionnelle d'un signal de mesure associé acquis pour une onde acoustique non-focalisée se propageant selon une direction de propagation associée,
- déterminer au moins un spectre bidimensionnel (P) à partir de la pluralité de tranches de profil (T), et
- déterminer ladite au moins une valeur représentative de l'intensité lumineuse (I) dans la zone d'observation (3), ladite valeur représentative étant fonction d'une transformation de Fourier bidimensionnelle inverse du spectre bidimensionnel (P).

## Patentansprüche

1. Akustooptisches Abbildungsverfahren zum Abbilden eines Beobachtungsbereichs (3) eines Mediums (2), wobei das Verfahren folgende Schritte aufweist
- einen Erfassungsschritt (100), bei dem eine Vielzahl von Messsignalen (S) erfasst wird, die einer Vielzahl von nicht fokussierten akustischen Wellen zugeordnet sind, die sich in nicht kollinearen Ausbreitungsrichtungen (θ) ausbreiten, wobei der Erfassungsschritt eine Vielzahl von Messvorgängen (150) aufweist, so dass während jedes Messvorgangs (150) der Vielzahl von Messvorgängen (150):
- in dem Beobachtungsbereich (3) eine nicht fokussierte akustische Welle erzeugt wird, die sich entlang einer Ausbreitungsrichtung (θ) ausbreitet;
- in den Beobachtungsbereich (3) eine einfallende Lichtwelle ausgesendet wird, um eine markierte Lichtwelle zu erzeugen, die mindestens eine akustooptische Komponente aufweist, deren Frequenz durch die nicht fokussierte akustische Welle verschoben wird;
- ein Messsignal (S) erfasst wird, das der nicht fokussierten akustischen Welle zugeordnet ist und Informationen über die markierte Lichtwelle enthält; und
- einen Bearbeitungsschritt (200), in dem mindestens ein Wert, der für eine Lichtintensität (I) in dem Beobachtungsbereich (3) repräsentativ ist, aus der Vielzahl von Messsignalen (S) bestimmt wird, wobei der Bearbeitungsschritt folgende Schritte aufweist:
- Bestimmen (210) einer Vielzahl von Teilprofilen (T), wobei jedes Teilprofil eine Funktion einer eindimensionalen Fouriertransformation eines zugehörigen Messsignals ist, das für eine nicht fokussierte akustische Welle erfasst wurde, die sich gemäß einer zugehörigen Ausbreitungsrichtung ausbreitet;
- Bestimmen eines zweidimensionalen Spektrums (P) aus der Vielzahl von Teilprofilen (T), und
- Bestimmen des mindestens einen repräsentativen Werts der Lichtintensität (I) im Beobachtungsbereich (3), wobei der repräsentative Wert eine Funktion einer inversen zweidimensionalen Fouriertransformation des zweidimensionalen Spektrums (P) ist.

2. Verfahren nach Anspruch 1, wobei die nicht fokussierte akustische Welle eine ebene akustische Welle ist.

3. Verfahren nach Anspruch 1, wobei die nicht fokussierte akustische Welle eine divergente akustische Welle ist.

4. Verfahren nach einem der Ansprüche 1 bis 3, wobei die Ausbreitungsrichtungen (θ) der mehreren nicht fokussierten akustischen Wellen einen Winkelsektor mit einem Winkel von mehr als 10 Grad abdecken.

5. Verfahren nach einem der Ansprüche 1 bis 4, wobei die Ausbreitungsrichtungen (θ) der mehreren nicht fokussierten akustischen Wellen durch einen Winkelabstand von mehr als 0,1 Grad, vorzugsweise mehr als 0,5 Grad, voneinander getrennt sind.

6. Verfahren nach einem der Ansprüche 1 bis 5, wobei die Ausbreitungsrichtungen (θ) der mehreren nicht fokussierten akustischen Wellen durch einen Winkelabstand von weniger als 45 Grad, vorzugsweise weniger als 20 Grad, voneinander getrennt sind.

7. Verfahren nach einem der Ansprüche 1 bis 6, wobei jedes Messsignal (S), das einer nicht fokussierten akustischen Welle zugeordnet ist, eine Zeitreihe von Lichtintensitätswerten aufweist, die Informationen über die akustooptische Komponente der markierten Lichtwelle enthält, die durch die nicht fokussierte akustische Welle in der Frequenz verschoben wird.

8. Verfahren nach einem der Ansprüche 1 bis 7, wobei jedes Messsignal (S), das einer nicht fokussierten akustischen Welle zugeordnet ist, mit einer Frequenz von mehr als 2 Megahertz, vorzugsweise mehr als zehn Megahertz, abgetastet wird.

9. Verfahren nach einem der Ansprüche 1 bis 8, wobei der Bearbeitungsschritt (200) die Durchführung einer inversen Radon-Transformation aufweist.

10. Verfahren nach einem der Ansprüche 1 bis 9, wobei der Bearbeitungsschritt (200) die Implementierung eines Spurbildungsalgorithmus aufweist.

11. Verfahren nach einem der Ansprüche 1 bis 10, wobei der Bearbeitungsschritt (200) die Implementierung eines Algorithmus zur Rückprojektion oder gefilterten Rückprojektion aufweist.

12. Verfahren nach einem der Ansprüche 1 bis 11, bei dem das zweidimensionale Spektrum (P) durch Neuberechnung der mehreren Teilprofile (T) in einem Fourierraum bestimmt wird, wobei jedes Teilprofil in Abhängigkeit von der Ausbreitungsrichtung (θ) einer nicht fokussierten akustischen Welle, die mit dem dem Teilprofil (T) zugeordneten Messsignal (S) zugeordnet ist, neu berechnet wird.

13. Verfahren nach einem der Ansprüche 1 bis 12, wobei ein Messvorgang (150) *n*-mal wiederholt wird, um n Messsignale (S) zu erfassen, die einer Ausbreitungsrichtung (θ) einer nicht fokussierten akustischen Welle zugeordnet sind, und wobei die n Messsignale insgesamt gemittelt werden, um ein der nicht fokussierten akustischen Welle zugeordnetes Messsignal zu bestimmen, das für den Bearbeitungsschritt (200) verwendet wird.

14. Verfahren nach einem der Ansprüche 1 bis 13, wobei der Messvorgang (150) für eine *n*-te Anzahl von Malen größer oder gleich 1 wiederholt wird.

15. System (1) zur akustooptischen Abbildung eines Beobachtungsbereichs (3) eines Mediums (2), wobei das System folgende Merkmale aufweist
- eine Erfassungsvorrichtung (11) zur Erfassung einer Vielzahl von Messsignalen (S), die einer Vielzahl von nicht fokussierten akustischen Wellen zugeordnet sind, die sich in nicht kollinearen Ausbreitungsrichtungen (θ) ausbreiten, wobei die Erfassungsvorrichtung folgende Merkmale aufweist:
- eine Anordnung von Wandlern (4), um in dem Beobachtungsbereich (3) eine Vielzahl von nicht fokussierten akustischen Wellen zu erzeugen, die sich in nicht kollinearen Ausbreitungsrichtungen (θ) ausbreiten,
- eine Lichtemissionsvorrichtung (8), um in den Beobachtungsbereich (3) mindestens eine einfallende Lichtwelle auszusenden, um eine Vielzahl von markierten Lichtwellen zu erzeugen, von denen jede mindestens eine akustooptische Komponente aufweist, die jeweils durch eine nicht fokussierte akustische Welle der Vielzahl von nicht fokussierten akustischen Wellen in der Frequenz verschoben ist,
- einen Detektor (9) zum Erfassen einer Vielzahl von Messsignalen (S), die der Vielzahl von nicht fokussierten akustischen Wellen zugeordnet sind und jeweils repräsentativ für jede markierte Lichtwelle der Vielzahl von markierten Lichtwellen sind; und
- eine Bearbeitungsvorrichtung (12), die so konfiguriert ist, dass sie mindestens einen Wert, der für eine Lichtintensität (I) in dem Beobachtungsbereich (3) repräsentativ ist, aus der Vielzahl von Messsignalen (S) bestimmt, die von der Erfassungsvorrichtung (11) erfasst werden, wobei die Bearbeitungsvorrichtung Mittel umfasst, die konfiguriert sind zum:
- Bestimmen (210) einer Vielzahl von Teilprofilen (T), wobei jedes Teilprofil eine Funktion einer eindimensionalen Fourier-Transformation eines zugehörigen Messsignals ist, das für eine nicht fokussierte akustische Welle erfasst wurde, die sich entlang einer zugehörigen Ausbreitungsrichtung ausbreitet,
- Bestimmen mindestens eines zweidimensionalen Spektrums (P) aus der Vielzahl von Teilprofilen (T), und
- Bestimmen des mindestens einen repräsentativen Werts der Lichtintensität (I) im Beobachtungsbereich (3), wobei der repräsentative Wert eine Funktion einer inversen zweidimensionalen Fourier-Transformation des zweidimensionalen Spektrums (P) ist.

## Claims

1. An acousto-optic imaging method for imaging an observation area (3) of a medium (2), the method comprising:
- an acquisition step (100) during which a plurality of measurement signals (S), associated with a plurality of non-focused acoustic waves propagating in non collinear directions (θ), is acquired, the acquisition step comprising a plurality of measurement operations (150) such that, in each measurement operation (150) of the plurality of measurement operations (150):
- a non-focused acoustic wave propagating in a propagation direction (θ) is generated in the observation area (3),
- an incident light wave is emitted in the observation area (3) in order to generate a marked light wave comprising at least one acoustic-optical component shifted in frequency by the non-focused acoustic wave,
- a measurement signal (S), associated with said non-focused acoustic wave, containing information about the marked light wave is acquired; and
- a processing step (200) during which at least one value representative of a light intensity (I) in the observation area (3) is determined from said plurality of measurement signals (S), the processing step comprising:
- determining (210) a plurality of profile slices (T), each profile slice being a function of a one-dimensional Fourier transform of an associated measurement signal acquired for a non-focused acoustic wave propagating along an associated propagation direction;
- determining a two-dimensional spectrum (P) from the plurality of profile slices (T), and
- determining said at least one value representative of a light intensity (I) in the observation area (3), said representative value being a function of a two-dimensional inverse Fourier transform of the two-dimensional spectrum (P).

2. The method according to claim 1, wherein the non-focused acoustic wave is an acoustic plane wave.

3. The method according to claim 1, wherein the non-focused acoustic wave is an acoustic diverging wave.

4. The method according to any one of claims 1 to 3, wherein the propagation directions (θ) of the plurality of non-focused acoustic waves cover an angular sector of angle greater than 10 degrees.

5. The method according to any one of claims 1 to 4, wherein the propagation directions (θ) of the plurality of non-focused acoustic waves are separated by an angular spacing greater than 0.1 degree, preferably greater than 0.5 degree.

6. The method according to any one of claims 1 to 5, wherein the propagation directions (θ) of the plurality of non-focused acoustic waves are separated by an angular spacing of less than 45 degrees, preferably less than 20 degrees.

7. The method according to any one of claims 1 to 6, wherein each measurement signal (S) associated with a non-focused acoustic wave comprises a time series of light intensity values containing information relating to the acoustic-optical component of the marked light wave shifted in frequency by the non-focused acoustic wave.

8. The method according to any one of claims 1 to 7, wherein each measurement signal (S) associated with a non-focused acoustic wave is sampled at a frequency greater than 2 megahertz, preferably greater than 10 megahertz.

9. The method according to any one of claims 1 to 8, wherein the processing step (200) comprises the implementation of an inverse Radon transform.

10. The method according to any one of claims 1 to 9, wherein the processing step (200) comprises the implementation of a beamforming algorithm.

11. The method according to any one of claims 1 to 10, wherein the processing step (200) comprises the implementation of a retroprojection or filtered retroprojection algorithm.

12. The method according to any one of claims 1 to 11, wherein the two-dimensional spectrum (P) is determined by readjusting in a Fourier space the plurality of profile slices (T), each profile slice being readjusted according to a propagation direction (θ) of a non-focused acoustic wave associated with the measurement signal (S) associated with the profile slice (T).

13. The method according to any one of claims 1 to 12, wherein a measurement operation (150) is repeated *n* times in order to acquire *n* measurement signals (S) associated with a propagation direction (θ) of a non-focused acoustic wave, and wherein said n measurement signals are averaged together to determine a measurement signal associated with said non-focused acoustic wave used for the processing step (200).

14. The method according to any one of claims 1 to 13, wherein the measurement operation (150) is repeated a number n of times that is greater than or equal to 1.

15. An Acousto-optic imaging system (1) for imaging an observation area (3) of a medium (2), the system comprising
- an acquisition device (11) for acquiring a plurality of measurement signals (S) associated with a plurality of non-focused acoustic waves propagating in non collinear directions (θ), the acquisition device comprising:
- a transducer array (4) for generating, in the observation area (3), a plurality of non-focused acoustic waves propagating in non collinear directions (θ),
- a light-emitting device (8) for emitting at least one incident light wave in the observation area (3), in order to generate a plurality of marked light waves each comprising at least one acoustic-optical component shifted in frequency respectively by one non-focused acoustic wave of said plurality of non-focused acoustic waves,
- a detector (9) for acquiring a plurality of measurement signals (S) associated with said plurality of non-focused acoustic waves and respectively representative of each marked light wave of said plurality of marked light waves; and
- a processing device (12) configured to determine at least one value representative of a light intensity (I) in the observation area (3), based on said plurality of measurement signals (S) acquired by the acquisition device (11), the processing device comprising means configured to:
- determine (210) a plurality of profile slices (T), each profile slice being a function of a one-dimensional Fourier transform of an associated measurement signal acquired for a non-focused acoustic wave propagating along an associated propagation direction;
- determine at least a two-dimensional spectrum (P) from the plurality of profile slices (T), and
- determine said at least one value representative of a light intensity (I) in the observation area (3), said representative value being a function of a two-dimensional inverse Fourier transform of the two-dimensional spectrum (P).
